# EUROPEAN PATENT APPLICATION

(11) **EP 4 516 317 A1**
(43) Date of publication of application: **05.03.2025**
(21) Application number: 22940328.2
(22) Date of filing: 21.12.2022
(51) Int. Cl.: A61K 45/00, A61P 43/00, A61P 17/16, A61Q 19/00, A61K 8/35, A61K 8/46, A61K 8/64, A61K 8/67, A61K 31/185

(54) **DERMATOLOGICAL TOPICAL AGENT COMPOSITION CONTAINING USEFUL COMPONENT-INCLUDING TAURINE CRYSTALS**

(30) Priority: 25.04.2022 JP 2022071711
(71) Applicant: Cosmed Pharmaceutical Co., Ltd., Kyoto-shi, Kyoto 601-8014 (JP)
(72) Inventor: MURAYAMA, Tomohiro, Kyoto-shi, Kyoto 601-8014 (JP); TANAKA, Hiroshi, Kyoto-shi, Kyoto 601-8014 (JP); HASEGAWA, Junya, Kyoto-shi, Kyoto 601-8014 (JP); QUAN, Ying-shu, Kyoto-shi, Kyoto 601-8014 (JP); KAMIYAMA, Fumio, Kyoto-shi, Kyoto 601-8014 (JP)
(74) Representative: Ter Meer Steinmeister & Partner
(86) International application number: PCT/JP2022/047061
(87) International publication number: WO 2023/210059

(57) **Abstract**

A new means of intradermal delivery of a useful component is provided. Disclosed are an external-use skin preparation composition comprising fine needlelike taurine crystals including a useful component in the crystals, and a method for producing a fine needlelike taurine crystal including a useful component in the crystal, the method comprising a step of growing a crystal in an environment in which an aqueous solution (A) containing taurine dissolved is mixed with or held in contact with a liquid substance (B) having low solubility of taurine, wherein the useful component is dissolved or dispersed in the aqueous solution (A) or the liquid substance (B), whereby the useful component is included in the crystal along with the growth of the crystal.

## Description

### TECHNICAL FIELD

The present invention relates to an external-use skin preparation composition containing a taurine crystal including a useful component. The present invention relates to a technique for incorporating a useful component during taurine recrystallization.

### BACKGROUND ART

Taurine is a compound having a chemical name of 2-aminoethanesulfonic acid, and is a sulfur-containing free amino acid highest in content in living cells among others. It is known that, unlike amino acids constituting proteins, taurine exhibits an action like vitamins and hormones, or exhibits various pharmacological actions including the cranial nervous system, the circulatory system, and the hepatobiliary system, but in recent years, taurine has been found to play an important role in moisture retention of the skin.

As a method for producing taurine, many production methods such as extraction from marine products and a plurality of organic synthesis methods are known. For example, Patent Document 1 describes a production method for obtaining taurine with high purity, and Patent Document 2 describes a production method for obtaining taurine with high yield.

As a technique for purifying taurine, Patent Document 3 describes a method for purifying taurine in which a solution containing taurine and an inorganic salt is brought into contact with a porous solid substance that exhibits a molecular sieving effect, the taurine is adsorbed to the porous solid substance to be separated from the inorganic salt, and then the taurine adsorbed to the porous solid substance is desorbed using an organic solvent which may contain water. In addition, as a means for purifying taurine with high yield, a technique of performing crystallization by adding alcohol is conventionally known. Namely, the technique is a method that involves, in purifying taurine from a hot water or warm water extraction solution of a natural product (marine product or the like) or a synthesis reaction end solution, precipitating taurine crystals in an aqueous alcohol solution, and then obtaining final taurine crystals by filtration and drying. Patent Document 4 describes a method for purifying taurine in which impurities are separated or removed by adding alcohol to a taurine-containing reaction liquid obtained through a production process and treating the taurine-containing reaction liquid. As a method for purifying crystals obtained, Patent Document 5 describes a method for purifying taurine in which crystals precipitated by crystallization are separated from a crystallization mother liquor, and then the crystals are treated with an aqueous alcohol solution containing hydrochloric acid, followed by a treatment with an aqueous alcohol solution free from hydrochloric acid.

On the other hand, as a technique for incorporating other components into a crystal, it has been studied to use components which are unstable in the state of an aqueous solution, are easily decomposed due to the influence of oxidation or temperature, and are difficult to stably incorporate, such as vitamins, amino acids, and peptides, while devising such that each component can be stabilized. Patent Document 6 describes that when calcium pantothenate and ascorbic acid are crystallized and mixed, stability is improved as compared with a mixture with non-crystallized calcium pantothenate. Patent Document 7 describes a method for obtaining a stable liquid preparation by adding a water-soluble vitamin in a crystalline state to an edible oil such as sunflower oil, corn oil, or cottonseed oil. Further, Patent Document 8 describes a lipstick composition containing a crystalline vitamin B3 compound.

### PRIOR ART DOCUMENTS

### PATENT DOCUMENTS

Patent Document 1: JP-A-2021-172652
Patent Document 2: JP-A-2021-063060
Patent Document 3: JP-A-2005-232026
Patent Document 4: JP-A-06-192209
Patent Document 5: JP-A-07-017943
Patent Document 6: JP-A-2005-325080
Patent Document 7: JP-A-2004-500392
Patent Document 8: JP-A-2002-536391

### SUMMARY OF THE INVENTION

### PROBLEMS TO BE SOLVED BY THE INVENTION

A production method and a purification method for obtaining taurine crystals with high purity and high yield are known, but conversely, it has not been known so far that taurine crystals are intentionally made to contain other active ingredients or skin useful components.

In addition, with respect to a technique for incorporating other components to a crystal, the composition to be used in the technique is a mixture of powders in a state in which water does not exist or a composition present in a dispersed state in oil, and the use thereof is limited to use in a non-aqueous system.

If a useful organic compound, a peptide, a dye, or the like can be stably contained in a water-soluble crystal, it is possible to color the crystal or to stably blend useful components in various preparations. It is an object of the present invention to provide a new means for intradermal delivery of a useful component.

### MEANS FOR SOLVING THE PROBLEMS

The present inventors have conducted intensive studies and, as a result, have found that creating a state with an extremely low solubility of taurine either by adding an aqueous solution containing taurine dissolved to a water-soluble organic solvent having a low solubility of taurine, such as an alcohol or a polyol, or by gently pouring the water-soluble organic solvent into the aqueous solution to form an interface separating the system into two phases, namely the aqueous taurine solution and the water-soluble organic solvent, and then advancing recrystallization make it possible to a fine needlelike taurine crystal and a hollow crystal is generated during the recrystallization. They have also found that a fine needlelike taurine crystal which carries a useful component such as a vitamin or a dye on its crystal surface or encloses the useful component within its hollow portion can be produced through, during the recrystallization, dissolving or dispersing the useful component in either or both of the aqueous taurine solution and the water-soluble organic solvent to be used. Furthermore, it has been found that there is no gritty feeling caused by crystals with an external-use skin preparation composition containing resulting crystals, and it is possible to provide a preparation to which a smooth application feeling of fine needlelike crystals is imparted.

The present invention is as follows.
[1] An external-use skin preparation composition comprising fine needlelike taurine crystals including a useful component in the crystals.
[2] The external-use skin preparation composition according to [1], wherein the fine needlelike taurine crystals are contained with at least a part thereof dispersed.
[3] The external-use skin preparation composition according to [1] or [2], wherein each of the fine needlelike taurine crystals contains a space, and a useful component is enclosed in the space.
[4] The external-use skin preparation composition according to [3], wherein a space volume in the fine needlelike taurine crystal accounts for 0.001% to 30% of a volume of the taurine crystal.
[5] The external-use skin preparation composition according to any one of [1] to [4], wherein the fine needlelike taurine crystals have a thickness of 150 micrometers or less and a length of 3000 micrometers or less.
[6] The external-use skin preparation composition according to any one of [1] to [5], wherein the useful component is one or two or more selected from the group consisting of vitamins and vitamin derivatives, hydroquinone and derivatives thereof, whitening components, anti-inflammatory components, antioxidant components, blood circulation promoting components, cell activating components, coenzymes and intermediate metabolites thereof, saccharides, plant extraction components, peptides, proteins, and dyes.
[7] The external-use skin preparation composition according to any one of [1] to [6], wherein an inclusion ratio of the useful component in the fine needlelike taurine crystals is 0.001% to 30%.
[8] The external-use skin preparation composition according to any one of [1] to [7], wherein the useful component is also dissolved or dispersed in an external-use skin preparation composition medium.
[9] A method for producing a fine needlelike taurine crystal including a useful component in the crystal, the method comprising a step of growing a crystal in an environment in which an aqueous solution (A) containing taurine dissolved is mixed with or held in contact with a liquid substance (B) having low solubility of taurine, wherein the useful component is dissolved or dispersed in the aqueous solution (A) or the liquid substance (B), whereby the useful component is included in the crystal along with the growth of the crystal.
[10] The production method according to [9], wherein the liquid substance (B) is a water-soluble organic solvent.
[11] The production method according to [10], wherein the water-soluble organic solvent is one or two or more selected from the group consisting of ethanol, isopropyl alcohol, ethylene glycol, 1,2-propanediol, 1,3-propanediol, 1,2-butanediol, 1,3-butanediol, isoprene glycol, diethylene glycol, dipropylene glycol, glycerin, diglycerin, 1,2-pentanediol, 1,2-hexanediol, cyclohexylglycerin, and n-hexylglycerin.
[12] The production method according to any one of [9] to [11], wherein a concentration of the useful component is 0.001% by mass or more in the aqueous solution (A) or the liquid substance (B).
[13] The production method according to any one of [9] to [12], wherein a concentration of taurine in the aqueous solution (A) is 3 to 28% by mass.
[14] The production method according to any one of [9] to [13], wherein the liquid substance (B) accounts for 50% by mass or less with respect to the entire amount of the aqueous solution (A).
[15] A method for producing an external-use skin preparation composition comprising a fine needlelike taurine crystal including a useful component in the crystal, the method comprising the following steps:
   a step of growing a crystal in an environment in which an aqueous solution (A) containing taurine dissolved is mixed with or held into contact with a liquid substance (B) having low solubility of taurine, wherein a useful component is dissolved or dispersed in the aqueous solution (A) or the liquid substance (B), whereby the useful component is included in the crystal along with the growth of the crystal; and
   blending the fine needlelike taurine crystal including the useful component in the crystal, which is obtained in the step of growing a crystal, into an external-use skin preparation composition.
[16] The production method according to [15], wherein a concentration of the useful component is 0.001% by mass or more in the aqueous solution (A) or the liquid substance (B).
[17] The production method according to [15] to [16], wherein a concentration of taurine in the aqueous solution (A) is 3 to 28% by mass.
[18] The production method according to any one of [15] to [17], wherein the liquid substance (B) accounts for 50% by mass or less with respect to the entire amount of the aqueous solution (A).
[19] The production method according to any one of [15] to [18], wherein the liquid substance (B) is a water-soluble organic solvent, and the water-soluble organic solvent is one or two or more selected from the group consisting of ethanol, isopropyl alcohol, ethylene glycol, 1,2-propanediol, 1,3-propanediol, 1,2-butanediol, 1,3-butanediol, isoprene glycol, diethylene glycol, dipropylene glycol, glycerin, diglycerin, 1,2-pentanediol, 1,2-hexanediol, cyclohexylglycerin, and n-hexylglycerin.

### EFFECT OF THE INVENTION

Since the external-use skin preparation composition of the present invention contains a fine needlelike taurine crystal including a useful component, the useful component can be stably retained in the composition. Since the taurine crystal in the present invention has been formed in a fine needlelike crystal by recrystallization, it is possible to insert the crystal into the stratum corneum without feeling a gritty feeling due to the crystal in use, then directly carry the useful component into the skin, and then supply the included component into the skin through dissolution of the crystal by moisture inside the skin.

### BRIEF DESCRIPTION OF THE DRAWINGS

FIG. 1 is an X-ray diffraction pattern of a fine needlelike taurine crystal obtained in Example 14. The upper graph is that of an astaxanthin-including fine needlelike taurine crystal, and the lower graph is that of a fine needlelike taurine crystal.
FIG. 2 is an optical micrograph of fine needlelike taurine crystals obtained in Example 3.
FIG. 3 is an optical micrograph of raw material taurine crystals.
FIG. 4 is an optical micrograph (50 magnifications) of fine needlelike taurine crystals obtained in Example 17.
FIG. 5 is a micrograph (50 magnifications under fluorescence) of fine needlelike taurine crystals obtained in Example 17.
FIG. 6 is an optical micrograph (200 magnifications) of a fine needlelike taurine crystal obtained in Example 17.
FIG. 7 is a micrograph (200 magnifications under fluorescence) of a fine needlelike taurine crystal obtained in Example 17.
FIG. 8 is a fluorescent photograph (50 magnifications) of a cross section of a skin 5 minutes after application.
FIG. 9 is a fluorescent photograph (50 magnifications) of a cross section of a skin 30 minutes after application.
FIG. 10 is a graph showing the measurement results of Example 19 and Comparative Example 7.

### MODE FOR CARRYING OUT THE INVENTION

The external-use skin preparation composition according to the present invention comprises fine needlelike taurine crystals including a useful component in the crystals. In the external-use skin preparation composition, it is preferable that the fine needlelike taurine crystals are contained with at least a part thereof dispersed.

The fine needlelike taurine crystals can be produced by mixing an aqueous solution (A) containing taurine dissolved with a liquid substance (B) having low solubility of taurine (for example, a water-soluble organic solvent), or bringing an aqueous solution (A) containing taurine dissolved and a liquid substance (B) having low solubility of taurine (for example, a water-soluble organic solvent) into contact with each other (for example, gently pouring the liquid substance (B) into the aqueous solution (A) to form an interface) to create a state with an extremely low solubility of taurine, and then advancing recrystallization (growing a crystal). The fine needlelike taurine crystals to be obtained each have a controlled crystal form. In addition, a hollow portion is formed in each of the fine needlelike taurine crystals when the crystals are produced, and this hollow portion is defined as a space volume.

Therefore, each of the fine needlelike taurine crystals has a space in the fine needlelike taurine crystal. The space volume of the fine needlelike taurine crystal preferably accounts for 0.001% to 30%, more preferably 0.1% to 30% of the volume of the taurine crystal. Here, the size of the space volume of the fine needlelike taurine crystal is calculated by analyzing a micrograph of the crystal.

The fine needlelike taurine crystals including a useful component in the crystals can be produced by dissolving or dispersing the useful component (an arbitrary organic compound component, peptide, protein, dye, or the like) in the aqueous solution (A) or the liquid substance (B) during the recrystallization of taurine. The fine needlelike taurine crystals including the useful component can be produced by making fine needlelike taurine crystals enclose or carry the useful component at the time of recrystallization. Herein, the term "enclose" refers to at least one of allow a useful component to enter into a taurine crystal lattice and include a useful component in a hollow portion (in a space) of a taurine crystal. In the former, the lattice constant increases as a taurine crystal expands. In the latter, the lattice constant does not increase due to the inclusion. Carrying means adsorbing or bonding to a surface of a taurine crystal. In the present invention, a fine needlelike taurine crystal enclosing or carrying a useful component, that is, a fine needlelike taurine crystal including a useful component in the crystal may be referred to as a useful-component-including fine needlelike taurine crystal. It is preferable that each of the fine needlelike taurine crystals contains a space, and a useful component is enclosed in the space.

Taurine as a raw material for use in the present invention is not particularly limited as long as it is conventionally used for pharmaceuticals, quasi-pharmaceutical products, or cosmetics, and includes both a synthetic product and an extract from a natural product.

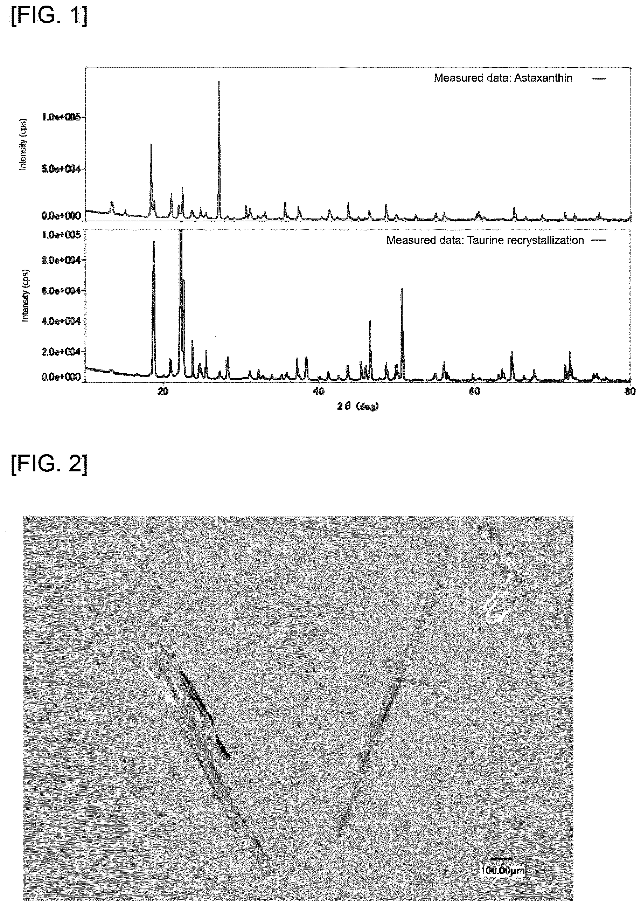

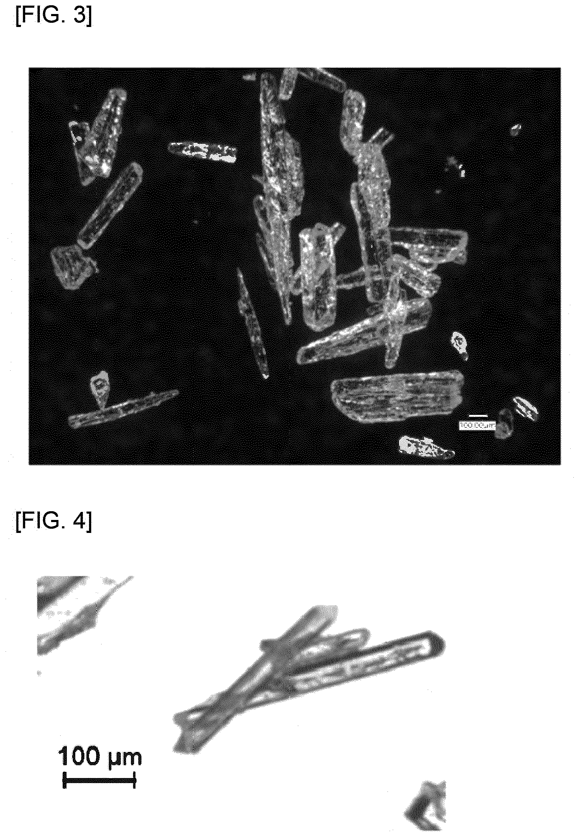

The fine crystal of taurine in the present invention is a needlelike crystal, but it may be a columnar crystal. Herein, the needlelike crystal refers to a shape in which the major axis of the crystal is 3 times or more the minor axis, and the columnar crystal refers to a shape in which the major axis of the crystal is less than 3 times the minor axis. The fine needlelike or columnar crystal of taurine in the present invention is a crystal form that can be distinguished from a massive crystal of the raw material taurine by microscopic observation. One example of fine needlelike crystals is shown in FIG. 2, and one example of massive crystals is shown in FIG. 3. Hereinafter, the taurine recrystallization used in the present invention is referred to as a fine needlelike taurine crystal.

The size of the fine needlelike taurine crystal is preferably 150 micrometers or less in thickness and 3000 micrometers or less in length, more preferably 5 to 150 micrometers in thickness and 100 to 3000 micrometers in length, still more preferably 10 to 100 micrometers in thickness and 100 to 2000 micrometers in length, and most preferably 10 to 60 micrometers in thickness and 150 to 2000 micrometers in length from the viewpoint of imparting a smooth application feeling without leaving a gritty feeling when the fine needlelike taurine crystal is used with incorporation in an external-use skin preparation composition.

Herein, the thickness is the minor axis of the crystal, and the length is the major axis of the crystal. The thickness and the length of a crystal can be measured using a digital microscope (for example, "Digital Microscope VHX-7000" manufactured by KEYENCE Corporation).

A crystal form more than 150 micrometers in thickness and more than 3000 micrometers in length (for example, taurine crystals and crystalline taurine powders which are commercially available as raw materials) is unpreferable because a gritty foreign matter feeling remains on the skin when applied.

In the present invention, fine needlelike taurine crystals may contain fine particle crystals less than 5 micrometers in thickness or less than 100 micrometers in length, but in particular, it is desirable not to contain crystals more than 150 micrometers in thickness. In the present invention, recrystallization conditions may be set such that needlelike crystals having a thickness exceeding 150 micrometers are not generated.

The specific recrystallization (crystal growth) condition is a step of growing a crystal under an environment in which the aqueous solution (A) containing taurine dissolved is mixed with or held in contact with the liquid substance (B) having low solubility of taurine.

The liquid substance (B) having low solubility of taurine, which is used for recrystallization (crystal growth), may be, but is not particularly limited to, an organic solvent, and examples thereof include polar organic solvents such as acetone, methyl ethyl ketone, dimethyl sulfoxide, dimethylformamide, ethyl acetate, isopropyl myristate, methyl heptyl laurate, and jojoba oil; nonpolar organic solvents such as dodecane, isododecane, liquid paraffin, squalane, hydrogenated polyisobutene, and olefin oligomers; alcohols such as ethanol and 1-propanol; and polyhydric alcohols such as 1,3-butanediol, glycerin, and 1,2-pentanediol. Among them, the liquid substance (B) is preferably a liquid substance (water-soluble organic solvent) having a solubility in water at 25°C of 1% by mass or more because the addition thereof has a high effect of lowering the solubility of taurine. Specifically, the liquid substance (B) is preferably ethanol, 1-propanol, 2-propanol, 1-butanol, 2-butanol, ethylene glycol, diethylene glycol, 1,2-propanediol, 1,3-propanediol, 1,2-butanediol, 1,3-butanediol, isoprene glycol, diethylene glycol, dipropylene glycol, glycerin, diglycerin, 1,2-pentanediol, 1,2-hexanediol, acetone, methyl ethyl ketone, dimethyl sulfoxide, dimethylformamide, cyclohexylglycerin, or n-hexylglycerin. From the viewpoint of using the obtained fine needlelike crystals as a component of an external-use skin preparation, the liquid substance (B) is most preferably ethanol, isopropyl alcohol, ethylene glycol, 1,2-propanediol, 1,3-propanediol, 1,2-butanediol, 1,3-butanediol, isoprene glycol, diethylene glycol, dipropylene glycol, glycerin, diglycerin, 1,2-pentanediol, 1,2-hexanediol, cyclohexylglycerin, or n-hexylglycerin. One of these solvents may be used, or a mixed solvent composed of two or more of them may be used.

A case of failing to use the liquid substance (B) is unpreferable because the resulting crystal form is not a fine needlelike crystal and when the resulting crystals are blended in an external-use skin preparation composition, a gritter sense of use is afforded.

The concentration of taurine in the aqueous solution (A) containing taurine dissolved to be used for recrystallization is preferably 3% by mass or more from the viewpoint of forming fine needlelike crystals when mixed with a solvent having low solubility of taurine, and the concentration is preferably 28% by mass or less from the viewpoint of the solubility of taurine in water. A concentration of less than 3% by mass is not realistic in terms of cost and yield because with such a concentration, crystals are not precipitated unless the liquid substance (B) is added in an amount of 100% by mass or more with respect to the aqueous solution (A), and a sufficient amount of crystals cannot be obtained. If the concentration is more than 28% by mass, the step of recrystallization is not realistic because it is difficult to dissolve taurine even when the mixture is heated to 80°C or higher. For these reasons, the concentration of taurine in the aqueous solution (A) containing taurine dissolved is preferably 5 to 20% by mass, more preferably 6 to 18% by mass, and most preferably 7 to 15% by mass.

To the aqueous solution (A) containing taurine dissolved to be used for recrystallization, one or two or more water-soluble organic solvents having low solubility of taurine may be added as the liquid substance (B) for the purpose of lowering the saturation concentration. As the water-soluble organic solvent in this case, glycerin, 1,3-propanediol, ethanol, isopropyl alcohol, ethylene glycol, 1,2-propanediol, 1,2-butanediol, 1,3-butanediol, isoprene glycol, diethylene glycol, dipropylene glycol, diglycerin, 1,2-pentanediol, 1,2-hexanediol, cyclohexylglycerin, n-hexylglycerin, and the like are preferable. The concentration of the liquid substance (B) (water-soluble organic solvent) is preferably 50% by mass or less, more preferably 30% by mass or less, and most preferably 25% by mass or less with respect to the total amount of the aqueous solution (A). A concentration exceeding 50% by mass is not realistic in terms of cost and yield because with such a concentration, the solubility of taurine becomes excessively low and a sufficient amount of crystals cannot be obtained.

The useful component included in the fine needlelike taurine crystals in the present invention is not particularly limited. Examples of the useful component include vitamins and vitamin derivatives, hydroquinone and derivatives thereof, whitening components, anti-inflammatory components, antioxidant components, blood circulation promoting components, cell activating components, coenzymes and intermediate metabolites thereof, saccharides, plant extraction components, peptides, proteins, and dyes.

Examples of the vitamins and vitamin derivatives, hydroquinone and derivatives thereof, whitening components, anti-inflammatory components, antioxidant components, blood circulation promoting components, cell activating components, coenzymes and intermediate metabolites thereof include nicotinamide, glycyrrhizic acid salts, tranexamic acid, L-ascorbic acid 2-glucoside, sodium ascorbyl phosphate, glyceryl ascorbic acid, bisglyceryl ascorbic acid, 3-glyceryl ascorbic acid, hexyl 3-glyceryl ascorbic acid, myristyl 3-glyceryl ascorbic acid, 3-lauryl glyceryl ascorbic acid, arbutin, kojic acid, allantoin, cyanocobalamin, riboflavin, pyridoxine salts, glucosylhesperidin, ascorbyl tetrahexyldecanoate, 3-O-ethylascorbic acid, tocopherol acetate, sodium copper chlorophyllin, folic acid, hydroquinone, ubiquinone, pyrroloquinolinequinone, topaquinone, tryptophan-tryptophylquinone, lysine tyrosylquinone, cysteinyl-tryptophan quinone, thiamine diphosphate, pantothenic acid, biotin, adenosine triphosphate, uridine diphosphate glucose, ribonucleotide, deoxynucleotide, nicotinamide mononucleotide, β-carotene, and astaxanthin.

The saccharides include D-glycerylaldehyde, dihydroxyacetone, D-erythrose, D-erythrulose, D-treose, erythritol, L-arabinose, D-xylose, L-lyxose, D-arabinose, D-ribose, D-ribulose, D-xylulose, L-xylulose, D-glucose, D-talose, D-bsicose, D-galactose, D-fructose, L-galactose, L-mannose, D-tagatose, aldoheptose, heplose, octulose, etc., 2-deoxy-D-ribose, 6-deoxy-L-galactose, 6-deoxy-L-mannose, D-glucosamine, D-galactosamine, sialic acid, aminouronic acid, muramic acid, etc., D-glucuronic acid, D-mannuronic acid, L-guluronic acid, D-galacturonic acid, L-iduronic acid sorbitol, sucrose, mannitol, fructose, raffinose, maltose, xylitol, maltitol, Palatinose, arabinose, maltotriose, sucrose, erythritol, glucose, starch-decomposed sugar, guntianose, umbelliferose, lactose, planteose, isolychnoses, trehalose, lychnoses, unbilicin, stachyose velvascose, hydrolyzed hydrogenated starch, and glyceryl glucoside.

Examples of the plant extraction component include components derived from vegetables such as Asian ginseng, Angelica keiskei, mountain arnica, gingko, fennel, turmeric, Isodonis japonicus, Dutch oak, chamomile, Roman chamomile, Daucus carota sativa, gentian, burdock, rice, Japanese hawthorn, shiitake mushroom, ginger, English hawthorn, juniper, Cnidium officinale Makino, swertia herb, thyme, clove, citrus unshiu, chili pepper, angelica root, peach kernel, spruce, carrot, garlic, butcher's broom, grape, peony, horse chestnut, lemon balm, yuzu, coix, green tea, rosemary, rose hip, citrus unshiu, angelica, spruce, peach, apricot, walnut, corn, golden chamomile, ichthammol, cantharides tincture, and cepharanthine.

The peptides include (arginine/lysine) polypeptide, 3-ascorbyl carbonyl dipeptide, acetyl cyclohexapeptide, acetyl tetrapeptide, acetyl tetrapeptide, acetyl tetrapeptide, acetyl tetrapeptide, acetyl hexapeptide, acetyl hexapeptide, acetyl heptapeptide, acetyl pentapeptide, oligopeptide, caffeoyl tripeptide, capryloyl dipeptide, capryloyl synthetic human nonapeptide, dipeptide, dipeptide diaminobutyroyl benzylamide diacetate, decapeptide, tetrapeptide, trifluoroacetyl tripeptide, copper tripeptide-1, nonapeptide, heptapeptide palmitate, palmitoyl octapeptide, palmitoyl oligopeptide, palmitoyl dipeptide, palmitoyl dipeptide-5 diaminohydroxybutyrate, palmitoyl dipeptide-5 diaminobutyroyl hydroxythreonine, palmitoyl tetrapeptide, rh-Oligopeptide, rh-Polypeptide, sh-Nonapeptide, N-prolyl palmitoyl tripeptide acetate, and hexanoyl dipeptide-3 norleucine acetate.

The proteins include almond protein, rice protein, wheat protein, wheat germ protein, soybean protein, corn gluten protein, and nacreous protein.

The dyes includes natural dyes and synthetic dyes. Examples of the natural dyes include gardenia yellow, carthamus yellow, turmeric oleoresin curcumin, monascus yellow, marigold color, monascus color, gardenia red, carthamus red, tomato color, cochineal extract carminic acid, perilla color, red cabbage color, red radish color, purple sweet potato color, grape skin color, cacao color, caramel color, and gardenia blue. Examples of the synthetic pigments include organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404; Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1.

The fine needlelike taurine crystals may include one of or two or more of the useful components.

The useful component is not limited to be a water-soluble component, and may be an oil-soluble component. In addition, even a component prone to be oxidized, especially, a component that has not heretofore been successfully blended due to odor change and discoloration with time lapse can be blended while being included in fine needlelike taurine crystals, so that the component can be allowed to stably exist. In addition, with fine needlelike taurine crystals in an external-use skin preparation composition, the external-use skin preparation composition is applied to the skin, and then the crystals dissolve and the included component dissolves, so that the enclosed component can be applied in a fresh state to the skin. Furthermore, it is possible to make taurine crystals include polymeric polysaccharides less likely to permeate the skin, peptides such as growth factors, and biological components such as proteins, and to directly pierce the skin with the needlelike taurine crystals in the composition to permeate those components in the skin.

As to the amount of the useful component to be included, the inclusion amount of the useful component accounting for the weight of the entire crystals can be indicated as an inclusion ratio. The inclusion ratio of the useful component in the fine needlelike taurine crystals is preferably 0.001 to 30% by mass, more preferably 0.001 to 25% by mass, and most preferably 0.001 to 20% by mass. An inclusion ratio of less than 0.001% by mass is unpreferable because it does not sufficiently afford the effect of the useful component included.

In order to successfully obtain fine needlelike taurine crystals having the above-described inclusion ratio, in the production of the fine needlelike taurine crystals, the useful component is preferably used in a state in which the useful component is dissolved or dispersed in the aqueous solution (A) containing taurine dissolved or the liquid substance (B) having low solubility of taurine. The concentration of the useful component is preferably 0.001% by mass or more in the aqueous solution (A) or the liquid substance (B) from the viewpoint of the amount of the enclosed component incorporated into the taurine crystals. A concentration of less than 0.001% by mass is unpreferable because it cannot be confirmed that the enclosed component is contained. The upper limit of the concentration of the useful component is not particularly limited.

The fine needlelike taurine crystal containing a useful component in the crystal may be filtered, dried, and then blended to an external-use skin preparation composition, or the solution in which the crystal has been precipitated may be blended as it is. The useful component not only is in the fine needlelike taurine crystal but also may be dissolved or dispersed in an external-use skin preparation composition medium. In this case, the useful component in the fine needlelike taurine crystal and the useful component dissolved or dispersed in the external-use skin preparation composition medium may be either the same or different.

The external-use skin preparation composition of the present invention can be used as an external-use skin preparation composition mainly for cosmetics and quasi-pharmaceutical products. The dosage form of the external-use skin preparation composition of the present invention is not particularly limited, and examples thereof include a liquid preparation, a lotion preparation, a cosmetic oil preparation, a milky lotion preparation, a cream preparation, an aqueous gel preparation, and an ointment preparation. These preparations can be prepared by conventional methods.

For a liquid preparation, a lotion preparation, a milky lotion preparation, a cream preparation, and an aqueous gel preparation, it is possible to prepare a preparation while maintaining the taurine concentration in the aqueous phase at a saturation concentration or higher and maintaining the useful-component-including fine needlelike taurine crystals.

The cosmetic oil preparation and the ointment preparation can be prepared by blending useful-component-including fine needlelike taurine crystals which have been collected by filtration, and then dried.

In addition, to the external-use skin preparation composition of the present invention, one or two or more components usually used in preparations such as cosmetics, quasi-pharmaceutical products, and pharmaceutical products for external use, that is, water, oil agents, surfactants, gelators, powders, alcohols, water-soluble polymers, film-forming agents, resins, UV protection agents, inclusion compounds, antibacterial agents, fragrances, deodorants, salts, pH adjusters, fresheners, animal/microorganism-derived extracts, plant extracts, blood circulation promoters, astringents, antiseborrheic drugs, whitening agents, anti-inflammatory agents, moisturizers, saccharification agents, keratolytic agents, enzymes, hormones, and vitamins may be appropriately added as long as the effects of the present invention are not impaired.

The water contained in the external-use skin preparation composition of the present invention is not particularly limited, and examples thereof include purified water, ion-exchanged water, and tap water.

Examples of a water-soluble alcohol include lower alcohols, polyhydric alcohols, polyhydric alcohol polymers, divalent alcohol alkyl ethers, dihydric alcohol alkyl ethers, dihydric alcohol ether esters, glycerin monoalkyl ethers, sugar alcohols, monosaccharides, oligosaccharides, polysaccharides, and derivatives of the foregoing.

Examples of the lower alcohols include ethanol, propanol, isopropanol, isobutyl alcohol, and t-butyl alcohol.

Examples of the polyhydric alcohols include dihydric alcohols (for example, dipropylene glycol, 1,3-butylene glycol, ethylene glycol, trimethylene glycol, 1,2-butylene glycol, tetramethylene glycol, 2,3-butylene glycol, pentamethylene glycol, 2-butene-1,4-diol, hexylene glycol, and octylene glycol), trihydric alcohols (for example, glycerin and trimethylolpropane), tetrahydric alcohols (for example, diglycerin and pentaerythritol, such as 1,2,6-hexanetriol), pentahydric alcohols (for example, xylitol and triglycerin), hexahydric alcohols (for example, sorbitol and mannitol), polyhydric alcohol polymers (for example, diethylene glycol, dipropylene glycol-triethylene glycol, polypropylene glycol, tetraethylene glycol, diglycerin-triglycerin, tetraglycerin, and polyglycerin), divalent alcohol alkyl ethers (for example, ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, and ethylene glycol dibutyl ether), dihydric alcohol alkyl ethers (for example, diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether triethylene glycol monomethyl ether triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, and dipropylene glycol butyl ether), dihydric alcohol ether esters (for example, ethylene glycol monomethyl ether acetate, ethylene glycol monoethyl ether acetate, ethylene glycol monobutyl ether acetate, ethylene glycol monophenyl ether acetate, ethylene glycol diadibate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, and propylene glycol monophenyl ether acetate), glycerin monoalkyl ethers (for example, xyl alcohol, selachyl alcohol, and batyl alcohol), sugar alcohols (for example, maltotriose, mannitol, sucrose, erythritol, glucose, fructose, starch-decomposed sugar, maltose, and starch sugar hydrogenated alcohol), glysolid, tetrahydrofurfuryl alcohol, POE-tetrahydrofuryl alcohol, POP-butyl ether, POP-POE-butyl-tertripolyoxypropylene glycerin ether, POP-glycerin ether, POP-glycerin ether phosphoric acid, POP-POE-pentaerythritol ether, and polyglycerin.

Examples of monosaccharides include triose (for example, D-glyceryl aldehyde, and dihydroxyacetone); tetrose (for example, D-erythrose, D- erythrulose, D-threose, and erythritol); pentose (for example, L-arabinose, D-xylose, L-lyxose, D-arabinose, D-ribose, D-ribulose, D-xylulose, and L-xylulose); hexose (for example, D-glucose, D-talose, D-psicose, D-galactose, D-fructose, L-galactose, L-mannose, and D-tagatose); heptose (for example, aldoheptose, and heplose); octose (for example, octulose); deoxy sugar (for example, 2-deoxy-D-ribose, 6-deoxy-L- galactose, and 6-deoxy-L-mannose); amino sugar (for example, D-glucosamine, D-galactosamine, sialic acid, aminouronic acid, and muramic acid); and uronic acid (for example, D-glucuronic acid, D-mannuronic acid, L-guluronic acid, D-galacturonic acid, and L-iduronic acid).

Examples of the oligosaccharide include sucrose, guntianose, umbelliferose, lactose, planteose, isolychnoses, α,α-trehalose, raffinose, lychnoses, umbilicin, and stachyose verbascoses.

Examples of the polysaccharide include cellulose, quince seed, starch, galactan, dermatan sulfate, glycogen, gum arabic, heparan sulfate-tragacanth gum, keratan sulfate, chondroitin, xanthan gum, guar gum, dextran, keratosulfate, locust bean gum, and succinoglucan.

Examples of the anti-inflammatory agent include plant-derived components, allantoin and derivatives thereof, glycyrrhetinic acid and derivatives thereof, glycyrrhizic acid and salts or derivatives thereof, salicylic acid derivatives, aminocaproic acid, and azulene and derivatives thereof.

Examples of the gelator (thickener) include gum arabic, carrageenan, gum karaya, gum tragacanth, carob gum, quince seed (marmelo), casein, dextrin, gelatin, sodium pectinate, sodium araginate, methyl cellulose, ethyl cellulose, CMC, hydroxyethyl cellulose, hydroxypropyl cellulose, PVA, PVM, PVP, sodium polyacrylate, carboxyvinyl polymer, locust bean gum, guar gum, tamarint gum, dialkyldimethylammonium sulfate cellulose, xanthan gum, aluminum magnesium silicate, bentonite, hectorite, AlMg silicate (Veegum), laponite, and silicic anhydride.

Examples of the natural water-soluble polymer include plant-based polymers (for example, gum arabic, gum tragacanth, galactan, guar gum, carob gum, gum karaya, carrageenan, pectin, agar, quince seed (marmelo), algae colloid (brown algae extract), starch (rice, corn, potato, wheat), and glycyrrhizic acid); microorganism-based polymers (for example, xanthan gum, dextran, succinoglycan, and pullulan); and animal-based polymers (for example, collagen, casein, albumin, and gelatine).

Examples of semi-synthetic water-soluble polymers include starch-based polymers (for example, carboxymethyl starch and methyl hydroxypropyl starch), cellulose-based polymers (methyl cellulose, ethyl cellulose, methyl hydroxypropyl cellulose, hydroxyethyl cellulose, cellulose sodium sulfate, hydroxypropyl cellulose, carboxymethyl cellulose, sodium carboxymethyl cellulose, crystalline cellulose, and cellulose powder), and alginic acid-based polymers (for example, sodium alginate and propylene glycol alginate) .

Examples of synthetic water-soluble polymers include vinyl base polymers (for example, polyvinyl alcohol, polyvinyl methyl ether, polyvinylpyrrolidone, and carboxyvinyl polymers); polyoxyethylene base polymers (for example, polyethylene glycol 20,000, 40,000, and 60,000); acrylic polymers (for example, sodium polyacrylate, polyethyl acrylate, and polyacrylamide); polyethyleneimine; and cationic polymers.

Examples of the moisturizer include chondroitin sulfate, hyaluronic acid, mucoitin sulfate, charonic acid, atelocollagen, cholesteryl 12-hydroxystearate, sodium lactate, bile salts, DL-pyrrolidone carboxylate, short-chain soluble collagen, diglycerin (EO)PO adduct, chestnut rose extract, yarrow extract, and melilot extract.

Examples of the whitening agents (whitening components) include tranexamic acid, ascorbic acid and salts thereof, vitamin C such as ascorbic acid derivatives (sodium ascorbate phosphate ester, magnesium ascorbate phosphate ester, ascorbyl tetra2-hexyldecanoate, 2-O-ethylascorbic acid, 3-O-ethylascorbic acid, ascorbyl glucoside, etc.), arbutin, kojic acid, placenta, ellagic acid, nicotinamide, hydroquinone, linoleic acid and derivatives thereof.

Examples of the keratolytic agent (keratin softening component) include lactic acid, salicylic acid, gluconic acid, glycolic acid, citric acid, malic acid, fruit acid, phytic acid, urea, and sulfur.

Examples of the anti-aging component include hydrolyzed soybean protein, retinoids (retinol and derivatives thereof, retinoic acid, retinal, and so on), kinetin, adenosine, NMN (nicotinamide mononucleotide), AMP (adenosine monophosphate), ADP (adenosine diphosphate), ATP (adenosine triphosphate), ursolic acid, turmeric extract, sphingosine derivatives, and mevalonolactone.

Examples of the anti-saccharification agent (anti-saccharification component) include plant extracts such as Buddleja axillaris leaf extract, evening primrose oil, fruit or fruit juice of amla or an extract thereof, L-arginine, L-lysine, hydrolyzed casein, hydrolyzable tannin, and carnosine.

Examples of the blood circulation promoter (blood circulation promoting component) include components derived from vegetables such as Asian ginseng, Angelica keiskei, mountain arnica, gingko, fennel, Isodonis japonicus, Dutch oak, chamomile, Roman chamomile, Daucus carota sativa, gentian, burdock, rice, Japanese hawthorn, shiitake mushroom, ginger, English hawthorn, juniper, Cnidium officinale Makino, swertia herb, thyme, clove, citrus unshiu, chili pepper, angelica root, peach kernel, spruce, carrot, garlic, butcher's broom, grape, peony, horse chestnut, lemon balm, yuzu, coix, green tea, rosemary, rose hip, citrus unshiu, angelica, spruce, peach, apricot, walnut, corn, golden chamomile, ichthammol, cantharides tincture, and cepharanthine; gamma oryzanol, tocopherol nicotinate, and glucosyl hesperidin.

Examples of polyphenols include flavonoid-based polyphenols such as curcuminoids, flavanones, stilpenoids, polymethoxyflavonoids, flavonols, xanthonoids, chalcones, lignoids, flavanols, and isoflavones.

Examples of a metal ion sequestrant include 1-hydroxyethane-1,1-diphosphonic acid, tetrasodium 1-hydroxyethane-1,1-diphosphonate, disodium edetate, trisodium edetate, tetrasorium edetate, sodium citrate, sodium polyphosphate, sodium metaphosphate, gluconic acid, phosphoric acid, citric acid, ascorbic acid, succinic acid, edetic acid, and trisodium hydroxyethyl ethylenediamine triacetate.

Examples of the UV protection agents (water-soluble ultraviolet absorbers) include benzophenone-based ultraviolet absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate salts, 4-phenylbenzophenone, 2-ethylhexyl-4'-phenyl-benzophenone-2-carboxylate, 2-hydroxy-4-n-octoxybenzophenone, and 4-hydroxy-3-carboxybenzophenone; benzimidazole-based ultraviolet absorbers such as phenylbenzimidazole-5-sulfonic acid and salts thereof, phenylene-bis-benzimidazole-tetrasulfonic acid and salts thereof; 3-(4'-methylbenzylidene)-d,1-camphor, 3-benzylidene-d,1-camphor, urocanic acid, and ethyl urocanate.

Examples of the powders (powder components) include inorganic powders (for example, talc, kaolin, mica, sericite, muscovite, phlogopite, synthetic mica, red mica, biotite, permiculite, magnesium carbonate, calcium carbonate, aluminum silicate, barium silicate, calcium silicate, magnesium silicate, strontium silicate, metal tungstates, magnesium, silica, zeolite, barium sulfate, calcined calcium sulfate (calcined gypsum), calcium phosphate, fluorapatite, hydroxyapatite, ceramic powder, metal soap (for example, zinc myristate, and calcium palmitate, aluminum stearate), and boron nitride); organic powders (for example, polyamide resin powder (nylon powder), polyethylene powder, polymethyl methacrylate powder, polystyrene powder, copolymer resin powder of styrene and acrylic acid, benzoguanamine resin powder, polytetrafluoroethylene powder, and cellulose powder); inorganic white pigments (for example, titanium dioxide and zinc oxide); inorganic red pigments (for example, iron oxide (red iron oxide) and iron titanate); inorganic brown pigments (for example, γ-iron oxide); inorganic yellow pigments (for example, yellow iron oxide and loess); inorganic black pigments (for example, black iron oxide and low valence titanium oxides); inorganic violet pigments (for example, mango violet and cobalt violet); inorganic green pigments (for example, chromium oxide, chromium hydroxide, and cobalt titanate); inorganic blue pigments (for example, ultramarine blue and iron blue); pearl pigments (for example, titanium oxide coated mica, titanium oxide coated bismuth oxychloride, titanium oxide coated talc, colored titanium oxide coated mica, bismuth oxychloride, and argentine); metal powder pigments (for example, aluminum powder, copper powder, or the like); organic pigments such as zirconium, barium or aluminum lake (for example, organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404; Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1); and natural pigments (for example, chlorophyll and β-carotene).

Examples of the vitamins include vitamins A, B1, B2, B6, C, and E and derivatives thereof, pantothenic acid and derivatives thereof, and biotin.

Examples of antioxidants include tocopherols, dibutylhydroxytoluene, butylhydroxyanisole, and gallate esters.

Examples of the pH adjusters include buffers such as lactic acid-sodium lactate, citric acid-sodium citrate, and succinic acid-sodium succinate.

Examples of the oil agents (oil phase components) include liquid oils, solid fats, waxes, hydrocarbon oils, higher fatty acids, synthetic ester oils, and silicone oils which are commonly used in cosmetics and quasi-pharmaceutical products. These oil phase components and the aqueous phase components described above can be combined with an appropriate surfactant to form a skin composition.

Examples of the liquid oils include avocado oil, camellia oil, turtle oil, macadamia nut oil, corn oil, mink oil, olive oil, rapeseed oil, egg yolk oil, sesame oil, persic oil, wheat germ oil, camellia kissi seed oil, castor oil, linseed oil, safflower oil, cotton seed oil, perilla oil, soybean oil, peanut oil, tea seed oil, kaya nut oil, rice bran oil, Chinese tung oil, Japanese tung oil, jojoba oil, germ oil, and triglycerol.

Examples of the solid fats include cocoa butter, coconut oil, horse fat, hardened coconut oil, palm oil, beef tallow, mutton fat, hardened beef tallow, palm kernel oil, lard, beef bone fat, Japan wax kernel oil, hardened oil, beef leg fat, Japan wax, and hardened castor oil.

Examples of the waxes include beeswax, candelilla wax, cotton wax, carnauba wax, bayberry wax, insect wax, whale wax, montan wax, rice bran wax, lanolin, kapok wax, lanolin acetate, liquid lanolin, corn wax, lanolin fatty acid isopropyl, hexyl laurate, hydrogenated lanolin, jojoba wax, hard lanolin, shellac wax, polyoxyethylene lanolin alcohol ether, polyoxyethylene lanolin alcohol acetate, polyoxyethylene cholesterol ether, polyethyleneglycol lanolin, polyoxyethylene hydrogenated lanolin alcohol ether, and cetyl palmitate.

Examples of the hydrocarbon oils include liquid paraffin, ozokerite, squalane, pristane, paraffin, ceresin. squalene, petrolatum, and microcrystalline wax.

Examples of the higher fatty acid include lauric acid, myristic acid, palmitic acid, stearic acid, behenic acid, oleic acid, undecylenic acid, tall oil acid, linoleic acid, linolenic acid, eicosapentaenoic acid (EPA), and docosahexaenoic acid (DHA).

Examples of the synthetic ester oils include cetyl octanoate, myristyl myristate, glyceryl tri(2-ethylhexanoate), pentaerythritol tetra (2-ethylhexanoate), dioctyl succinate, and tripropylene glycol dineopentanoate.

Examples of the silicone oils include chain polysiloxanes (for example, dimethylpolysiloxane, methylphenylpolysiloxane, and diphenylpolysiloxane); cyclic polysiloxanes (for example, octamethylcyclotetrasiloxane, decamethylcyclopentasiloxane, and docecamethylcyclohexasiloxane); silicon resins with three-dimensional network; silicon rubbers; modified polysiloxanes (for example, amino-modified polysiloxane, polyether-modified polysiloxane, alkyl-modified polysiloxane, and fluorine-modified polysiloxane); and acrylic silicones.

Examples of amino acids include glycine, alanine, valine, leucine, isoleucine, which are neutral amino acids, serine, threonine, which are aliphatic amino acids and are oxyamino acids, cysteine, cystine, methionine, which are aliphatic amino acids and are sulfur-containing amino acids, glutamic acid, aspartic acid, which are acidic amino acids, glutamine, asparagine, which are amino acids having an amide group, arginine, which is a basic amino acid, phenylalanine, tyrosine, which are aromatic amino acids, proline and oxyproline, which have an imino group.

The method for producing an external-use skin preparation composition of the present invention comprises the following steps:
a step of growing a crystal in an environment in which an aqueous solution (A) containing taurine dissolved is mixed with or held in contact with a liquid substance (B) having low solubility of taurine, wherein the useful component is dissolved or dispersed in the aqueous solution (A) or the liquid substance (B), whereby the useful component is included in the crystal along with the growth of the crystal; and
a step of blending the fine needlelike taurine crystal including the useful component in the crystal, which is obtained in the step of growing a crystal, into an external-use skin preparation composition.

An individual external-use skin preparation composition can be prepared by various production methods, for example, a method involving preparing a formulation while maintaining useful-component-including fine needlelike taurine crystals by keeping the taurine concentration in an aqueous phase during the production process at a saturated concentration or more, or a method involving blending useful-component-including fine needlelike taurine crystals which have been obtained through recrystallization, collected by filtration, and then dried. For details, the prescription examples of the following Examples can be referred to.

### EXAMPLES

Hereinafter, the present invention will be described in more detail by the following Examples. These Examples are merely examples for specifically describing the present invention, and the scope of the present invention is not limited to these Examples. Hereinafter, each blending amount in Examples and the like is in % by mass, and thickness and length are expressed in the unit of micrometer unless otherwise specified.

### Comparative Example 1

An aqueous solution (100 mL) containing 10% of taurine and 0.1% of cyanocobalamin was heated to 50°C until uniform dissolution, then the solution was allowed to cool to room temperature. After leaving the mixture at rest for 24 hours, the crystals precipitated were collected by filtration and dried, affording pink crystals.

### Comparative Example 2

An aqueous solution (100 mL) containing 10% of taurine, 0.1% of cyanocobalamin and 15mL of ethanol was heated to 50°C until uniform dissolution, then the solution was allowed to cool to room temperature. After leaving the mixture at rest for 24 hours, the crystals precipitated were collected by filtration and dried, affording pink crystals.

### Comparative Example 3

An aqueous solution (100 mL) containing 10% of taurine, 5.0% of nicotinamide and 15mL of ethanol was heated to 50°C until uniform dissolution, then the solution was allowed to cool to room temperature. After leaving the mixture at rest for 24 hours, the crystals precipitated were collected by filtration and dried, affording colorless, transparent crystals. The crystals obtained were analyzed by high performance liquid chromatography to confirm that nicotinamide was contained.

### Comparative Example 4

An aqueous solution (100 mL) containing 10% of taurine, 5.0% of nicotinamide and 15mL of ethanol was heated to 50°C until uniform dissolution, then the solution was cooled to 30°C with stirring with a spatula. The crystals precipitated were collected by filtration and dried, affording colorless, transparent crystals. The crystals obtained were analyzed by high performance liquid chromatography to confirm that nicotinamide was contained.

### Comparative Example 5

An aqueous solution (100 mL) containing 10% of taurine, 10% of glycerin, and 0.0008% of nicotinamide was heated to 50°C until uniform dissolution, then cooled to 30°C with stirring. Then, 15 mL of ethanol was slowly poured, and crystals precipitated at the interface separating the system into two phases were collected by filtration, washed with ethanol, and then dried. The crystals obtained were analyzed by high performance liquid chromatography, but nicotinamide was not detected.

### Comparative Example 6

An aqueous solution (100 mL) containing 2.5% of taurine was heated to 50°C until uniform dissolution, then the solution was cooled to 30°C with stirring. After leaving the mixture at rest, 80 mL of an ethanol solution containing 5% of nicotinamide was slowly poured, but no crystals were obtained.

### Example 1

An aqueous solution (100 mL) containing 10% of taurine and 0.1% of cyanocobalamin was heated to 60°C until uniform dissolution, and then cooled to 30°C with stirring. 1,3-Butanediol (15 mL) was added with strong stirring with a disper, and the precipitated crystals were collected by filtration, washed with ethanol, and then dried, affording pink crystals.

### Example 2

An aqueous solution (100 mL) containing 10% of taurine and 10% of glycerin was heated to 50°C until uniform dissolution, and then cooled to 30°C with stirring. An ethanol solution (15 mL) containing 1.0% of cyanocobalamin was added with strong stirring with a disper, and the precipitated crystals were collected by filtration, washed with ethanol, and then dried, affording pink crystals.

### Example 3

An aqueous solution (100 mL) containing 15% of taurine was heated to 50°C until uniform dissolution, then cooled to 40°C with stirring, and left at rest. Then, 15 mL of an ethanol solution containing 1% of cyanocobalamin heated to 40°C was slowly poured, and crystals precipitated at the interface separating the system into two phases were collected by filtration, washed with ethanol, and then dried, affording pink crystals.

### Example 4

An aqueous solution (100 mL) containing 10% of taurine was heated to 50°C until uniform dissolution, then cooled to 30°C with stirring, and left at rest. Then, 15 mL of an ethanol solution containing 5% of nicotinamide was slowly poured, and crystals precipitated at the interface separating the system into two phases were collected by filtration, washed with ethanol, and then dried, affording colorless, transparent crystals. The crystals obtained were analyzed by high performance liquid chromatography to confirm that nicotinamide was contained.

### Example 5

An aqueous solution (100 mL) containing 5% of taurine was heated to 50°C until uniform dissolution, then cooled to 30°C with stirring, and left at rest. Then, 15 mL of an ethanol solution containing 5% of 3-O-ethyl ascorbic acid was slowly poured, and crystals precipitated at the interface separating the system into two phases were collected by filtration, washed with ethanol, and then dried, affording colorless, transparent crystals. The crystals obtained were analyzed by high performance liquid chromatography to confirm that 3-O-ethyl ascorbic acid was contained.

### Example 6

An aqueous solution (100 mL) containing 20% of taurine and 3% of dipotassium glycyrrhizinate was heated to 70°C until uniform dissolution, then cooled to 50°C with stirring, and left at rest. Then, 15 mL of 1,3-propanediol heated to 50°C was slowly poured, and crystals precipitated at the interface separating the system into two phases were collected by filtration, washed with a 50% aqueous ethanol solution, and then dried, affording colorless, transparent crystals. The crystals obtained were analyzed by high performance liquid chromatography to confirm that dipotassium glycyrrhizinate was contained.

### Example 7

An aqueous solution (100 mL) containing 8% of taurine was heated to 50°C until uniform dissolution, then cooled to 30°C with stirring, and left at rest. Then, 15 mL of an ethanol solution containing 5% of L-ascorbic acid 2-glucoside was slowly poured, and crystals precipitated at the interface separating the system into two phases were collected by filtration, washed with ethanol, and then dried, affording colorless, transparent crystals. The crystals obtained were analyzed by high performance liquid chromatography to confirm that L-ascorbic acid 2-glucoside was contained.

### Example 8

An aqueous solution (100 mL) containing 25% of taurine and 5% of arbutin was heated to 80°C until uniform dissolution, and then left at rest. Then, 15 mL of 1,3-butylene glycol heated to 80°C was slowly poured, and crystals precipitated at the interface separating the system into two phases were collected by filtration, washed with ethanol, and then dried, affording colorless, transparent crystals. The crystals obtained were analyzed by high performance liquid chromatography to confirm that arbutin was contained.

### Example 9

An aqueous solution (100 mL) containing 10% of taurine was heated to 50°C until uniform dissolution, then cooled to 30°C with stirring, and left at rest. Then, 15 mL of an ethanol solution containing 10% ascorbyl tetrahexyldecanoate was slowly poured, and crystals precipitated at the interface separating the system into two phases were collected by filtration, washed with ethanol, and then dried, affording colorless, transparent crystals. The crystals obtained were analyzed by high performance liquid chromatography to confirm that ascorbyl tetrahexyldecanoate was contained.

### Example 10

An aqueous solution (100 mL) containing 15% of taurine and 0.01% of allantoin was heated to 60°C until uniform dissolution, then cooled to 40°C with stirring, and left at rest. Then, 15 mL of ethanol was slowly poured, and crystals precipitated at the interface separating the system into two phases were collected by filtration, washed with a 50% aqueous ethanol solution, and then dried, affording colorless, transparent crystals. The crystals obtained were analyzed by high performance liquid chromatography to confirm that allantoin was contained.

### Example 11

An aqueous solution (100 mL) containing 12% of taurine was heated to 50°C until uniform dissolution, then cooled to 30°C with stirring, and left at rest. Then, 15 mL of an ethanol solution containing 0.1% of tocopherol acetate was slowly poured, and crystals precipitated at the interface separating the system into two phases were collected by filtration, washed with ethanol, and then dried, affording colorless, transparent crystals. The crystals obtained were analyzed by high performance liquid chromatography to confirm that tocopherol acetate was contained.

### Example 12

An aqueous solution (100 mL) containing 10% of taurine and 0.5% of sodium copper chlorophyllin was heated to 50°C until uniform dissolution, then cooled to 30°C with stirring, and left at rest. Then, 15 mL of ethanol was slowly poured, and crystals precipitated at the interface separating the system into two phases were collected by filtration, washed with a 50% aqueous ethanol solution, and then dried, affording green crystals.

### Example 13

An aqueous solution (100 mL) containing 10% of taurine and 1% of gardenia blue was heated to 50°C until uniform dissolution, then cooled to 30°C with stirring, and left at rest. Then, 15 mL of ethanol was slowly poured, and crystals precipitated at the interface separating the system into two phases were collected by filtration, washed with a 50% aqueous ethanol solution, and then dried, affording blue crystals.

### Example 14

An aqueous solution (100 mL) containing 12% of taurine was heated to 50°C until uniform dissolution, then cooled to 35°C with stirring, and left at rest. Then, 15 mL of an ethanol solution containing 5% of ubidecarenone heated to 40°C was slowly poured, and crystals precipitated at the interface separating the system into two phases were collected by filtration, washed with ethanol heated to 40°C, and then dried, affording pale yellow needlelike crystals containing ubidecarenone.

### Example 15

An aqueous solution (100 mL) containing 10% of taurine was heated to 50°C until uniform dissolution, then cooled to 35°C with stirring, and left at rest. Then, 15 mL of an ethanol solution containing 3% of a β-carotene suspension (β-carotene 30% suspension: DSM Co., Ltd.) was slowly poured, and crystals precipitated at the interface separating the system into two phases were collected by filtration, washed with ethanol heated to 40°C, and then dried, affording pink needlelike crystals containing β-carotene.

### Example 16

An aqueous solution (100 mL) containing 8% of taurine was heated to 50°C until uniform dissolution, then cooled to 35°C with stirring, and left at rest. Then, 15 mL of an ethanol solution containing 3% of an astaxanthin solution (ASTAXANTHIN-5C: Oryza Oil & Fat Chemical Co., Ltd.) was slowly poured, and crystals precipitated at the interface separating the system into two phases were collected by filtration, washed with ethanol heated to 40°C, and then dried, affording orange needlelike crystals containing astaxanthin.

The crystals obtained in Comparative Examples 1 to 5 and Examples 1 to 16 were microscopically observed. The crystal form, the thickness and the length of the crystals were confirmed. Next, the sense of use when each crystal was blended in the milky lotion formulation given in Table 1 and the cosmetic oil formulation given in Table 2 was evaluated with the scores shown in Table 3. The results are shown in Tables 4 to 6.

With regard to the milky lotion preparations, the taurine concentration in a phase A was set to a saturated concentration at 25°C, a phase B was added to the phase A heated to 80°C while being mixed with a homomixer, the mixture was cooled to 25°C, a phase C was then mixed, and taurine crystal of a layer D was mixed with the composition obtained to prepare a milky lotion preparation containing crystals.

With regard to the cosmetic oil preparations, a phase A was heated to 100°C and mixed, then cooled to 25°C, and taurine crystals of a layer B were mixed to prepare a cosmetic oil preparation containing crystals.

**[Table 1]**

| | Component | % by mass |
|---|---|---|
| Phase A | Purified water | 61.81 |
| | 1,3-Propanediol | 15.00 |
| | Taurine | 6.00 |
| | Glycerin | 5.00 |
| | Pentylene glycol | 1.50 |
| | (Hydroxyethyl acrylate/Na acryloyldimethyltaurate) copolymer | 1.88 |
| | Isohexadecane | 1.13 |
| | Polysorbate 80 | 0.38 |
| Phase B | Squalane | 5.00 |
| Phase C | Phenoxyethanol | 0.30 |
| Phase D | Taurine crystals obtained in Comparative Examples and Examples | 2.00 |

**[Table 2]**

| | Component | % by mass |
|---|---|---|
| Phase A | Squalane | 62.90 |
| | Hydrogenated polydecene | 29.00 |
| | Hydrogenated (styrene/isoprene) copolymer | 7.00 |
| | Tocopherol | 0.10 |
| Phase B | Taurine crystals obtained in Comparative Examples and Examples | 1.00 |

**[Table 3]**

| | |
|---|---|
| 0 points | A strong gritty feel is always felt. |
| 2 points | A weak gritty feel is always felt. |
| 4 points | A weak gritty feel is often felt. |
| 6 points | A weak gritty feel is felt very rarely. |
| 8 points | A smooth feel is felt. |
| 10 points | A very smooth feel is felt. |

**[Table 4]**

| | Comparative Example 1 | Comparative Example 2 | Comparative Example 3 | Comparative Example 4 | Comparative Example 5 | Example 1 | Example 2 |
|---|---|---|---|---|---|---|---|
| Crystal form | Columnar | Columnar | Columnar | Columnar | Needlelike | Columnar | Columnar |
| Maximum particle size (thickness) µm | 1680 | 1320 | 1830 | 350 | 25 | 145 | 70 |
| Maximum particle size (length) µm | 6740 | 5820 | 6340 | 640 | 250 | 280 | 100 |
| Score of milky lotion preparation | 0.0 | 0.0 | 0.0 | 1.2 | 8.8 | 8.0 | 8.4 |
| Score of cosmetic oil preparation | 0.0 | 0.0 | 0.0 | 0.8 | 8.4 | 8.0 | 8.4 |

**[Table 5]**

| | Example 3 | Example 4 | Example 5 | Example 6 | Example 7 | Example 8 | Example 9 |
|---|---|---|---|---|---|---|---|
| Crystal form | Needlelike | Needlelike | Needlelike | Needlelike | Needlelike | Needlelike | Needlelike |
| Maximum particle size (thickness) µm | 30 | 25 | 20 | 30 | 40 | 20 | 20 |
| Maximum particle size (length) µm | 300 | 250 | 250 | 220 | 150 | 200 | 180 |
| Score of milky lotion preparation | 8.4 | 8.8 | 9.2 | 9.2 | 9.2 | 9.6 | 10.0 |
| Score of cosmetic oil preparation | 8.0 | 8.8 | 9.6 | 9.2 | 8.8 | 9.6 | 9.6 |

**[Table 6]**

| | Example 10 | Example 11 | Example 12 | Example 13 | Example 14 | Example 15 | Example 16 |
|---|---|---|---|---|---|---|---|
| Crystal form | Needlelike | Needlelike | Needlelike | Needlelike | Needlelike | Needlelike | Needlelike |
| Maximum particle size (thickness) µm | 20 | 25 | 25 | 40 | 20 | 30 | 30 |
| Maximum particle size (length) µm | 250 | 250 | 250 | 520 | 180 | 300 | 230 |
| Score of milky lotion preparation | 9.6 | 9.6 | 9.2 | 8.8 | 9.6 | 9.2 | 9.2 |
| Score of cosmetic oil preparation | 9.6 | 9.2 | 9.2 | 8.4 | 10.0 | 8.8 | 9.2 |

As shown in Tables 4 to 6, it was confirmed that the crystals obtained by the production method of the present invention formed fine needlelike or columnar crystals, and due to the control of the crystal size, no gritty feel was observed in the sense of use of the blended products, and the sense of use was clearly improved.

### Example 17

An aqueous solution (100 mL) containing 10% by mass of taurine and 10% by mass of glycerin was heated to 50°C until uniform dissolution, then cooled to 40°C with stirring, and left at rest. Then, 15 mL of an ethanol solution containing 5% by mass of uranine (fluorescent substance) heated to 40°C was slowly poured, and crystals precipitated at the interface separating the system into two phases were collected by filtration, washed with ethanol, and then dried, affording orange-yellow crystals. Micrographs of the crystals obtained are shown in FIGS. 4 to 7.

As shown in FIGS. 4 to 7, the shapes of the fine taurine crystals obtained were needlelike. The fine needlelike crystals had a maximum thickness of 30 micrometers and a maximum length of 230 micrometers.

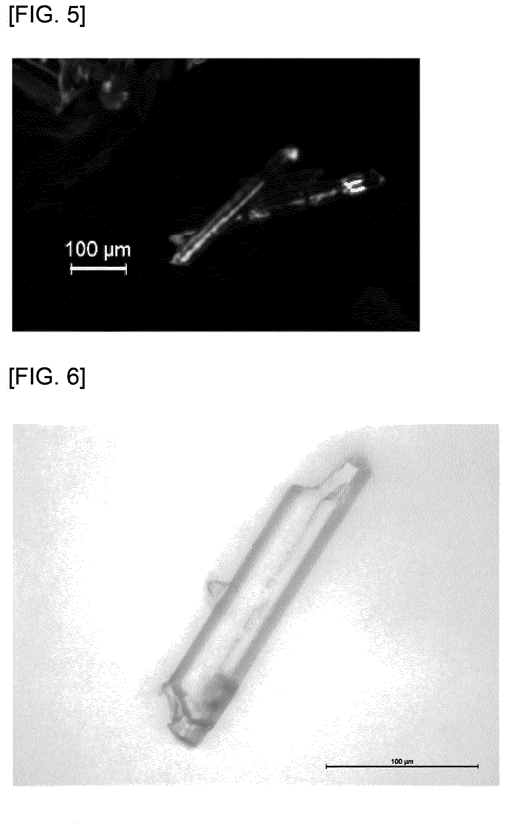

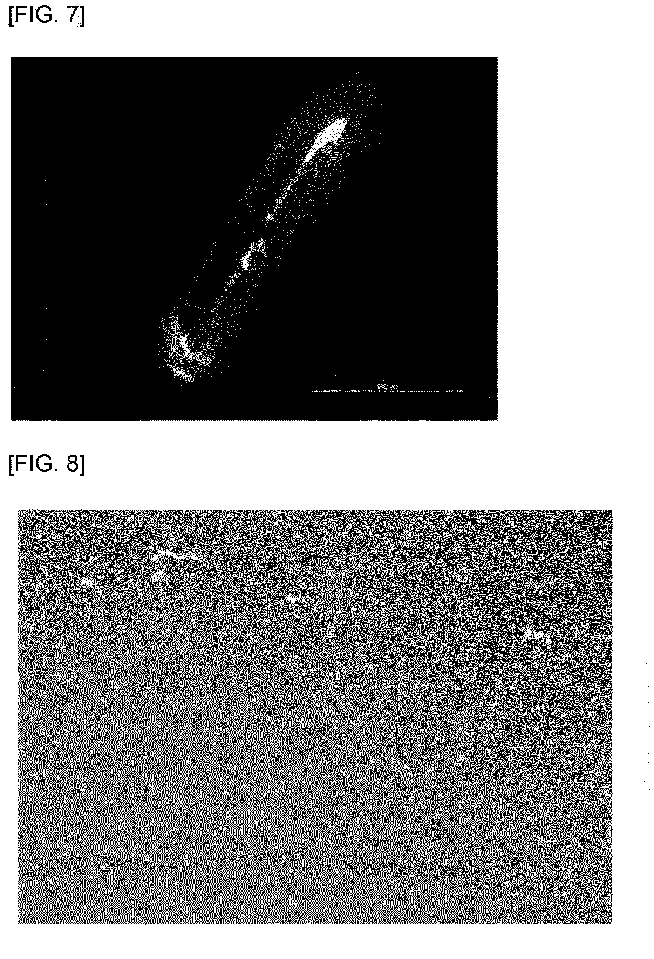

As shown in FIGS. 5 and 7, when a crystal obtained was observed with a fluorescence microscope, it was confirmed that uranine was enclosed in the crystal.

### Example 18

The following composition containing the crystals obtained in Example 17 was prepared, and the permeation of the enclosed component into the skin was evaluated.

### [Composition]

[A]

| | |
|---|---|
| Purified water | 81.60% by mass |
| Taurine | 8.00% by mass |
| Carboxyvinyl polymer | 0.20% by mass |

[B]

| | |
|---|---|
| Arginine | 0.20% by mass |

[C]
Fine needlelike crystal obtained in Example 17 10.0% by mass

The phase A heated to 80°C and uniformly dissolved was cooled to 35°C, the phase B was added with stirring, and the resulting mixture was stirred at 35°C for 10 minutes. The phase C was added thereto, and the mixture was stirred for 10 minutes to uniformly mix, affording an aqueous gel composition.

The composition obtained was applied to a human excised skin (thickness: 700 micrometers) (provided by U.S. Tissue Bank), and fluorescence observation of a skin cross section after 5 minutes was performed. As a result, it was confirmed that fine needlelike crystals including uranine stuck in the skin and reached the horny layer, the epidermis, and the dermis (FIG. 8).

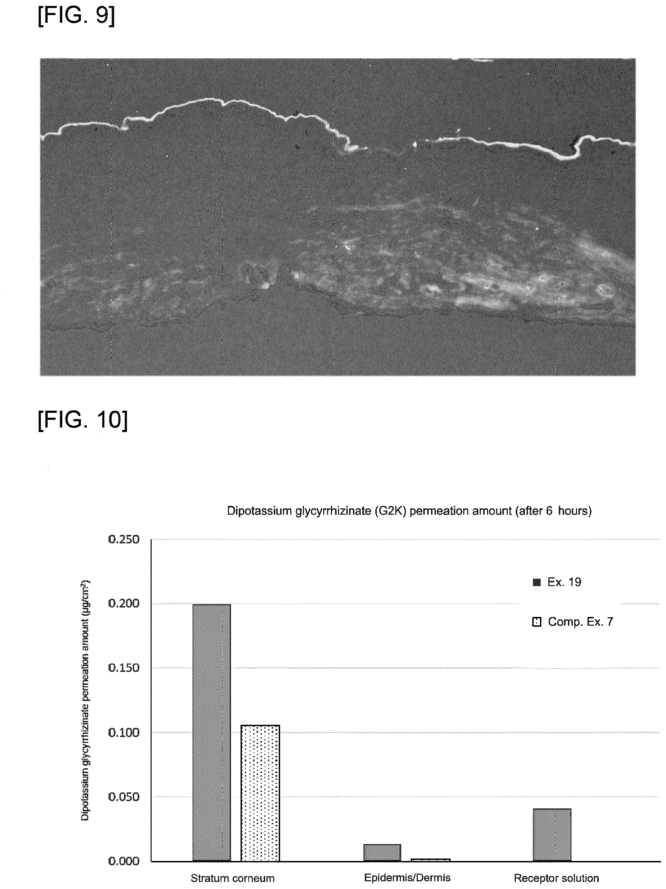

Furthermore, when the state after 30 minutes was observed, it was confirmed that crystals that had stuck in the skin were dissolved, and uranine was diffused in the skin (FIG. 9).

### Example 19 and Comparative Example 7

Fine needlelike crystals including dipotassium glycyrrhizinate were prepared, and the amount of dipotassium glycyrrhizinate permeated into the skin was measured.

An aqueous solution (100 mL) containing 10% by mass of taurine and 3% by mass of dipotassium glycyrrhizinate was heated to 60°C until uniform dissolution, then cooled to 40°C with stirring, and left at rest. Then, 15 mL of ethanol was slowly poured, and crystals precipitated at the interface separating the system into two phases were collected by filtration, washed with ethanol, and then dried, affording crystals.

The shape of the fine taurine crystals obtained was needlelike. The fine needlelike crystals had a maximum thickness of 30 micrometers and a maximum length of 180 micrometers.

Further, the crystals obtained were analyzed by liquid chromatography, and it was confirmed that 0.101% by mass of dipotassium glycyrrhizinate was contained.

A composition given in the following table containing 10% by mass of the fine needlelike crystals and a composition of Comparative Example in which dipotassium glycyrrhizinate was dissolved in the same concentration as that composition were prepared, and the amounts of dipotassium glycyrrhizinate permeated into the skin when the obtained compositions were applied to a human skin were compared.

**[Table 7]**

| | Example 19 | Comparative Example 7 |
|---|---|---|
| Purified water | 81.6% by mass | 99.9899% by mass |
| Taurine | 8.0% by mass | - |
| Carboxyvinyl polymer | 0.2% by mass | - |
| Arginine | 0.2% by mass | - |
| Dipotassium glycyrrhizinate | - | 0.0101% by mass |
| Dipotassium glycyrrhizinate-containing crystal of Example 19 | 10.0% by mass (0.0101% by mass in terms of dipotassium glycyrrhizinate) | - |

For the evaluation of the amount of permeation into the skin, a vertical Franz diffusion cell having an effective diameter of 2.0 centimeters, an area of 3.14 square centimeters, and a receptor fluid capacity of 2.4 mL was used, and each sample was applied to a human excised skin at 10.0 mg/square centimeter.

After 6 hours, the stratum corneum and the epidermis/dermis were separated from the sample skin by tape stripping, and dipotassium glycyrrhizinate contained in each of the stratum corneum, the epidermis/dermis, and the receptor solution was quantified by high performance liquid chromatography.

As shown in FIG. 10, the composition of Example 19 afforded a permeation amount of dipotassium glycyrrhizinate in the entire skin of about 2.5 times as compared with the composition of Comparative Example 7, and it was confirmed that, as a result of making the useful component to be included in the fine needlelike crystals, the useful component had permeated into the epidermis/dermis and the receptor liquid.

Formulation examples are shown below, but the present invention is not limited to these examples.

### [Skin lotion]

[A]

| | |
|---|---|
| Purified water | 71.60% by mass |
| Taurine | 7.80% by mass |
| Glycerin | 5.00% by mass |
| Diglycerin | 0.50% by mass |
| Betaine | 0.50% by mass |
| Phenoxyethanol | 0.30% by mass |

[B]

| | |
|---|---|
| Dipropylene glycol | 10.00% by mass |
| 1,2-Pentanediol | 2.50% by mass |

[C]

| | |
|---|---|
| (Acrylates/steareth-20 methacrylate) copolymer | 0.45% by mass |
| Purified water | 1.05% by mass |

[D]

| | |
|---|---|
| Crystal obtained in Example 1 | 0.30% by mass |

The phase A heated to 80°C and uniformly dissolved was cooled to 35°C, added to the phase B with stirring, and the resulting mixture was stirred at 35°C for 10 minutes. To the mixture was added the phase C, and the resulting mixture was stirred with a homomixer for 10 minutes to uniformly mix. Thereafter, crystals of the phase D were added thereto, and the resulting mixture was stirred for 5 minutes, affording a skin lotion composition.

The fine crystals of taurine in the composition had a needlelike shape and had a maximum thickness of 30 micrometers and a maximum length of 300 micrometers. The composition had a smooth sense of use, and a composition having a good sense of use was obtained.

### [Aqueous gel]

[A]

| | |
|---|---|
| Purified water | 71.40% by mass |
| Taurine | 8.00% by mass |
| Glycerin | 5.00% by mass |
| Raffinose | 0.50% by mass |
| Phenoxyethanol | 0.30% by mass |

[B]

| | |
|---|---|
| Dipropylene glycol | 10.00% by mass |
| 1,2-Pentanediol | 2.50% by mass |

[C]

| | |
|---|---|
| Polyacrylate crosspolymer-6 | 2.00% by mass |

[D]

| | |
|---|---|
| Crystal obtained in Example 14 | 0.30% by mass |

The phase A heated to 80°C and uniformly dissolved was cooled to 35°C, added to the phase B with stirring, and the resulting mixture was stirred at 35°C for 10 minutes to precipitate fine needlelike taurine crystals. To the mixture was added the phase C, and the resulting mixture was stirred with a homomixer for 10 minutes to uniformly mix. Thereafter, crystals of the phase D were added thereto, and the resulting mixture was stirred for 5 minutes, affording an aqueous gel composition.

The fine crystals of taurine in the composition had a needlelike shape and had a maximum thickness of 30 micrometers and a maximum length of 230 micrometers. The composition had a smooth sense of use, and a composition having a good sense of use was obtained.

### [Milky lotion]

[A]

| | |
|---|---|
| Purified water | 60.20% by mass |
| Taurine | 8.00% by mass |
| Glycerin | 5.00% by mass |

[B]

| | |
|---|---|
| 1,3-Propanediol | 15.00% by mass |
| 3-0-Ethylascorbic acid | 3.00% by mass |
| Pentylene glycol | 1.50% by mass |
| Phenoxyethanol | 0.30% by mass |

[C]

| | |
|---|---|
| (Hydroxyethyl acrylate/Na acryloyldimethyltaurate) copolymer | 0.75% by mass |
| Isohexadecane | 0.45% by mass |
| Polysorbate 80 | 0.15% by mass |
| Purified water | 0.65% by mass |

[D]

| | |
|---|---|
| Squalane | 5.00% by mass |

The phase A heated to 80°C and uniformly dissolved was cooled to 35°C, added to the phase B with stirring, and the resulting mixture was stirred at 35°C for 10 minutes to precipitate fine needlelike taurine crystals. To this was added the mixture of the phase C, and the resultant was stirred with a homomixer for 5 minutes to uniformly mix. Then, the phase D was added, and the resulting mixture was stirred with a homomixer for 10 minutes to emulsify, affording a milky lotion composition.

The fine crystals of taurine in the obtained composition had a needlelike shape and had a maximum thickness of 30 micrometers and a maximum length of 250 micrometers, and analysis by high-performance liquid chromatography confirmed that 3-O-ethylascorbic acid was contained. The composition had a smooth sense of use, and a composition having a good sense of use was obtained.

### [O/W type cream]

[A]

| | |
|---|---|
| Purified water | 63.00% by mass |
| Taurine | 8.00% by mass |
| Glycerin | 5.00% by mass |
| Nicotinamide | 5.00% by mass |

[B]

| | |
|---|---|
| 1,3-Propanediol | 15.00% by mass |
| Pentylene glycol | 1.50% by mass |
| Phenoxyethanol | 0.30% by mass |

[C]

| | |
|---|---|
| (Hydroxyethyl acrylate/Na acryloyldimethyltaurate) copolymer | 0.75% by mass |
| Polysorbate 80 | 0.15% by mass |
| Purified water | 0.65% by mass |

[D]

| | |
|---|---|
| Squalane | 0.65% by mass |

The phase A heated to 80°C and uniformly dissolved was cooled to 35°C, added to the phase B with stirring, and the resulting mixture was stirred at 35°C for 10 minutes to precipitate fine needlelike taurine crystals. To this was added the mixture of the phase C, and the resultant was stirred with a homomixer for 5 minutes to uniformly mix. Then, the phase D was added, and the resulting mixture was stirred with a homomixer for 10 minutes to emulsify, affording an O/W type cream composition.

The fine crystals of taurine in the obtained composition had a needlelike shape and had a maximum thickness of 30 micrometers and a maximum length of 250 micrometers, and analysis by high-performance liquid chromatography confirmed that nicotinamide was contained. The composition had a smooth sense of use, and a composition having a good sense of use was obtained.

### [W/O type cream]

[A]

| | |
|---|---|
| Purified water | 32.30% by mass |
| Taurine | 7.00% by mass |
| Glycerin | 5.00% by mass |
| L-Ascorbic acid 2-glucoside | 2.00% by mass |

[B]

| | |
|---|---|
| 1,3-Propanediol | 9.00% by mass |
| 1,2-Hexanediol | 0.70% by mass |
| Phenoxyethanol | 0.30% by mass |

[C]

| | |
|---|---|
| Isopropyl myristate | 33.70% by mass |
| Jojoba oil | 5.00% by mass |
| Polyglyceryl polyricinoleate-6 | 3.25% by mass |
| Polyglyceryl isostearate-2 | 1.00% by mass |
| Disteardimonium hectorite | 0.75% by mass |

The phase A heated to 80°C and uniformly dissolved was cooled to 35°C, added to the phase B with stirring, and the resulting mixture was stirred at 35°C for 10 minutes to precipitate fine needlelike taurine crystals. This mixed liquid was added to the homogeneously mixed phase C over 5 minutes while applying a homomixer, and then stirred and emulsified with the homomixer for 10 minutes, affording a W/O type cream composition.

The fine crystals of taurine in the obtained composition had a needlelike shape and had a maximum thickness of 25 micrometers and a maximum length of 200 micrometers, and analysis by high-performance liquid chromatography confirmed that L-ascorbic acid 2-glucoside was contained. The composition had a smooth sense of use, and a composition having a good sense of use was obtained.

### [Cosmetic oil]

[A]

| | |
|---|---|
| Cetyl 2-ethylhexanoate | 63.40% by mass |
| Hydrogenated polydecene | 29.00% by mass |
| Hydrogenated (styrene/isoprene) copolymer | 7.00% by mass |
| Tocopherol | 0.10% by mass |

[B]

| | |
|---|---|
| Fine needlelike taurine crystal obtained in Example 11 | 0.50% by mass |

The phase A was heated to 100°C and stirred until becoming uniform. The mixture was cooled to 35°C, the B phase was added thereto, and the resulting mixture was stirred to uniformly disperse, affording a cosmetic oil composition.

The fine crystals of taurine in the composition had a needlelike shape and had a maximum thickness of 40 micrometers and a maximum length of 520 micrometers. The composition had a smooth sense of use, and a composition having a good sense of use was obtained.

### Test Example 1

For the various useful component-including taurine produced as described above, a test to demonstrate that a useful component was included in taurine crystals was performed. The result of X-ray diffraction of the astaxanthin-including needlelike taurine crystals produced in Example 14 is described below.

As a control, needlelike crystals of taurine alone were produced through the following process.

An aqueous solution (100 mL) containing 10% of taurine was heated to 50°C until uniform dissolution, then cooled to 30°C with stirring, and left at rest. Then, 15 mL of ethanol was slowly poured, and crystals precipitated at the interface separating the system into two phases were collected by filtration, washed with ethanol, and then dried, affording colorless, transparent crystals.

X-ray diffraction of a needlelike taurine crystal and an astaxanthin-including needlelike taurine crystal was performed using an X-ray diffractometer manufactured by Rigaku Corporation, model SmartLab. A CuKα ray (wavelength = 0.154 nm) was used as an X-ray, an output was 45 kV and 200 mA, HyPix-3000 was used as a detector, and measurement was performed at a scanning speed of 10°/min within a 2θ range of 10 to 80° by the 2θ/θ measurement method.

The obtained X-ray diffraction pattern is shown in FIG. 1. The upper chart is the diffraction pattern derived from an astaxanthin-including taurine crystal, and the lower chart is the diffraction pattern derived from a taurine crystal.

From the above results, the astaxanthin-including taurine crystal and the taurine crystal do not exhibit the same diffraction pattern. The diffraction angles of the 111 plane and the 200 plane are summarized in the following Table 8. Specifically, the lattice constants were calculated from the following data using the Bragg's equation for the expansions of the 111 plane and the 200 plane (a decrease in 2θ indicates an expansion of the lattice spacing).

**[Table 8]**

| | 2θ | |
|---|---|---|
| Diffraction plane | (111) | (200) |
| Taurine crystal | 22.25° | 22.60° |
| Astaxanthin-including taurine crystal | 22.02° | 22.48° |

| | | |
|---|---|---|
| Expansion due to astaxanthin on 111 plane = 0.004 nm Expansion due to astaxanthin on 200 plane = 0.002 nm | | |

One example of an optical micrograph of the crystals obtained according to the present invention is shown in FIG. 2. This crystal is that obtained in Example 3. One example of a micrograph of a crystal of the raw material taurine is shown in FIG. 3. As the raw material taurine, taurine in the Pharmacopoeia of Japan (manufactured by Shoka Pharmacy Co., Ltd.) was used.

## Claims

1. An external-use skin preparation composition comprising fine needlelike taurine crystals including a useful component in the crystals.

2. The external-use skin preparation composition according to claim 1, wherein the fine needlelike taurine crystals are contained with at least a part thereof dispersed.

3. The external-use skin preparation composition according to claim 1 or 2, wherein each of the fine needlelike taurine crystals contains a space, and a useful component is enclosed in the space.

4. The external-use skin preparation composition according to claim 3, wherein a space volume in the fine needlelike taurine crystal accounts for 0.001% to 30% of a volume of the taurine crystal.

5. The external-use skin preparation composition according to any one of claims 1 to 4, wherein the fine needlelike taurine crystals have a thickness of 150 micrometers or less and a length of 3000 micrometers or less.

6. The external-use skin preparation composition according to any one of claims 1 to 5, wherein the useful component is one or two or more selected from the group consisting of vitamins and vitamin derivatives, hydroquinone and derivatives thereof, whitening components, anti-inflammatory components, antioxidant components, blood circulation promoting components, cell activating components, coenzymes and intermediate metabolites thereof, saccharides, plant extraction components, peptides, proteins, and dyes.

7. The external-use skin preparation composition according to any one of claims 1 to 6, wherein an inclusion ratio of the useful component in the fine needlelike taurine crystals is 0.001% to 30%.

8. The external-use skin preparation composition according to any one of claims 1 to 7, wherein the useful component is also dissolved or dispersed in an external-use skin preparation composition medium.

9. A method for producing a fine needlelike taurine crystal including a useful component in the crystal, the method comprising a step of growing a crystal in an environment in which an aqueous solution (A) containing taurine dissolved is mixed with or held in contact with a liquid substance (B) having low solubility of taurine, wherein the useful component is dissolved or dispersed in the aqueous solution (A) or the liquid substance (B), whereby the useful component is included in the crystal along with the growth of the crystal.

10. The production method according to claim 9, wherein the liquid substance (B) is a water-soluble organic solvent.

11. The production method according to claim 10, wherein the water-soluble organic solvent is one or two or more selected from the group consisting of ethanol, isopropyl alcohol, ethylene glycol, 1,2-propanediol, 1,3-propanediol, 1,2-butanediol, 1,3-butanediol, isoprene glycol, diethylene glycol, dipropylene glycol, glycerin, diglycerin, 1,2-pentanediol, 1,2-hexanediol, cyclohexylglycerin, and n-hexylglycerin.

12. The production method according to any one of claims 9 to 11, wherein a concentration of the useful component is 0.001% by mass or more in the aqueous solution (A) or the liquid substance (B).

13. The production method according to any one of claims 9 to 12, wherein a concentration of taurine in the aqueous solution (A) is 3 to 28% by mass.

14. The production method according to any one of claims 9 to 13, wherein the liquid substance (B) accounts for 50% by mass or less with respect to the entire amount of the aqueous solution (A).

15. A method for producing an external-use skin preparation composition comprising a fine needlelike taurine crystal including a useful component in the crystal, the method comprising the following steps:
a step of growing a crystal in an environment in which an aqueous solution (A) containing taurine dissolved is mixed with or held into contact with a liquid substance (B) having low solubility of taurine, wherein a useful component is dissolved or dispersed in the aqueous solution (A) or the liquid substance (B), whereby the useful component is included in the crystal along with the growth of the crystal; and
blending the fine needlelike taurine crystal including the useful component in the crystal, which is obtained in the step of growing a crystal, into an external-use skin preparation composition.

16. The production method according to claim 15, wherein a concentration of the useful component is 0.001% by mass or more in the aqueous solution (A) or the liquid substance (B).

17. The production method according to claim 15 or 16, wherein a concentration of taurine in the aqueous solution (A) is 3 to 28% by mass.

18. The production method according to any one of claims 15 to 17, wherein the liquid substance (B) accounts for 50% by mass or less with respect to the entire amount of the aqueous solution (A).

19. The production method according to any one of claims 15 to 18,
wherein the liquid substance (B) is a water-soluble organic solvent, and
the water-soluble organic solvent is one or two or more selected from the group consisting of ethanol, isopropyl alcohol, ethylene glycol, 1,2-propanediol, 1,3-propanediol, 1,2-butanediol, 1,3-butanediol, isoprene glycol, diethylene glycol, dipropylene glycol, glycerin, diglycerin, 1,2-pentanediol, 1,2-hexanediol, cyclohexylglycerin, and n-hexylglycerin.
